# EUROPEAN PATENT SPECIFICATION

(11) **EP 0 941 471 B1**
(45) Date of publication and mention of the grant of the patent: **02.10.2002**
(21) Application number: 97948081.1
(22) Date of filing: 04.12.1997
(51) Int. Cl.: G01N 25/02, B22D 2/00, C22C 33/08

(54) **METHOD FOR JUDGING THE PROPERTIES OF MOLTEN CAST IRON**
VERFAHREN ZUM BEURTEILEN DER EIGENSCHAFTEN VON GESCHMOLZENEM GUSSEISEN
PROCEDE PERMETTANT D'ESTIMER LES PROPRIETES DE LA FONTE A L'ETAT FONDU

(30) Priority: 04.12.1996 SE 9604478; 21.11.1997 SE 9704276
(43) Date of publication of application: 15.09.1999
(73) Proprietor: SinterCast AB, 100 55 Stockholm (SE)
(72) Inventor: SHAO, Sten, SinterCast Technologies AB, S-641 30 Katrineholm (SE); HOLLINGER, Bertil, SinterCast Technologies AB, S-641 30 Katrineholm (SE)
(74) Representative: Berg, Sven Anders
(86) International application number: SE9702031
(87) International publication number: WO98025133

(56) References cited:
- EP-A- 0 138 499
- WO-A-86/01755
- WO-A-92/06810
- GB-A- 2 283 325
- GB-A- 2 300 916

## Description

The present invention relates to an improved method for predicting the microstructure with which a certain cast iron will solidify. The invention also relates to an apparatus for carrying out the method

### Background of the invention

WO86/01755 disclosed a method for producing compacted graphite cast iron by using thermal analysis. A sample is taken from a bath of molten cast iron and this sample is permitted to solidify during 0.5 to 10 minutes. The temperature is recorded simultaneously by two temperature responsive means, one of which is arranged in the centre of the sample and the other in the immediate vicinity of the vessel wall. So-called cooling curves representing temperature as a function of time are recorded for each of the two temperature responsive means. According to this document it is then possible to determine the necessary amount of structure-modifying agents that has to be added to the melt in order to obtain the desired microstructure.

WO92/06809 describes a specific method for evaluating the cooling curves obtained by the method of WO86/01755. According to this document thermal analysis is carried out in a sample vessel coated with a material consuming the active form of the structure-modifying agent. This material may comprise oxides of Si, Mn, Fe, K and Na. An early plateau in the cooling curve (which, because of the coating often is found close to the vessel wall) means that flake graphite crystals have precipitated close to the temperature-responsive means recording the curve. A skilled person can, by studying the curves and by using calibration data, determine whether any structure-modifying agent has to be added to the melt in order to obtain compacted graphite case iron.

WO92/06810 describes a method for producing spheroidal cast iron where a thermal analysis is carried out in a sample vessel coated with the same material as in WO92/06809.

Accordingly, it is known to predict in which form a certain cast iron melt will solidify by using thermal analysis and coated sample vessels. However, the prediction is difficult to carry out for low concentrations of structure-modifying agent near the borderline between grey cast iron and compacted graphite cast iron. However, it is often desirable to work with cast iron melts containing a minimal amount of structure-modifying agents. Hence, there is need for prediction methods giving accurate and easily construable results, also in this concentration region.

EP 0 327 237 discloses a method of testing the magnesium content of a melt in connection with production of spheroidal cast iron. A molten sample of magnesium-treated iron is introduced into a sample vessel containing additives of tellurium and either sulphur or selenium. The sample is then allowed to cool and the temperature is continuously monitored.

A basic idea in the method of EP 0 327 237 is to remove all active magnesium from the sample before carrying out thermal analysis. Then the melt can either solidify with graphitic or carbidic arrest.

The reason for adding sulphur or selenium together with tellurium is that, in the presence of magnesium, tellurium alone cannot prevent the iron from solidifying with a graphitic eutectic structure. Therefore, the addition of selenium or sulphur is just sufficient to completely neutralize a predetermined threshold percentage of magnesium. If the magnesium content is above the threshold percentage, the melt will solidify as graphitic iron.

EP 0 327 237 also suggests that the method can be modified to control the magnesium content of compacted graphite cast irons. In that case the document states that two separate thermal analysis with different amounts of added sulfur are needed.

GB 2,300,916 relates to a method for judging the properties of molten cast iron by using three sampling vessels and recording cooling curves, one cup containing tellurium, the second being free of additive and the third having ferrosilicon as additive. For the second cup, the difference between maximum and minimum eutectic temperatures is measured. A local maximum temperature is the tellurium cup is compared with eutectic temperatures for other cups. The types of cast iron predicted are of flaky graphite types A to E.

EP 0 138 499 discloses a molten metal sampler with tellurium additive.

GB 2,283,325 discloses a method for thermal analysis of molten cast iron. The method consists of the steps of: a) obtaining a first cooling curve of the molten cast iron using a clean cup; b) obtaining a second cooling curve of the molten cast iron using a second cup in which small amounts (e.g. 0.5 to 0.3 % by weight) of tellurium are contained; and c) comparing the first cooling curve with the second cooling curve. The two curves are only compared in respect of recalscence. A recalscence larger than zero relates to compacted graphite cast iron (CGI) or spheroidal graphite cast iron (SGI). Such a comparison is satisfying when it is desirable to check if structure-modifying agent had been added or not before pouring while producing SGI or CGI. However, the method is not sufficiently accurate in the border region between CGI and grey iron. When producing CGI and carrying out a thermal analysis according to the inaccurate method disclosed in GB 2,283,325, there is a large risk that graphite flake patched are formed locally in the casting, due to a slightly insufficient concentration of structure-modifying agent in the molten cast iron. As the presence of flake patches in the microstructure reduces the tensile strength and elastic modules of the iron by 20-50 %, this is a serious problem.

### Summary of the invention

Now it has turned out that unexpectedly good predictions of the microstructure with which a certain cast iron melt will solidify can be performed by carrying out a method as defined in claim 1.

### Detailed description of the invention

The present invention is based on the discovery that easily construable results are obtained when carrying out thermal analysis of a sample of molten cast iron that has been treated with an appopriate amount of at least one substance neutralizing the structure-modifying agent, which substance is tellurium. Hence, the present method does not require a highly qualified staff and it is very suitable for automation by computer.

The term "cooling curve" as utilized herein refers to graphs representing the temperature as a function of time during solidification of a cast iron melt.

The term "sample vessel" as disclosed herein refers to a sample container which, when used for thermal analysis, is filled with a sample of molten metal. The temperature of the molten metal is then recorded in a suitable way.

The term "structure-modifying agent" as disclosed herein refers to an agent affecting the microstructure of cast iron, which agent is magnesium.

The term "tellurium cup" as disclosed herein refers to a sample vessel coated with tellurium or containing a tellurium blob. The tellurium cup may also contain conventional inoculating agents.

The term "FeSi cup" as disclosed herein refers to a sample vessel coated with FeSi, or containing a FeSi blob. The FeSi cup may also contain conventional inoculating agents.

The term "clean cup" as disclosed herein refers to a sample vessel without any coating, or with an inert coating.

The term "sampling device" as disclosed herein refers to a device comprising a sample vessel equipped with at least one sensor for thermal analysis, said sensor(s) being intended to be immersed in the solidifying metal sample during analysis, and a means for filling the sample vessel with molten metal.

The term "neutralizing agent" as disclosed herein refers to an agent neutralizing the active form of the structure-modifying agent and is tellurium.

The term "CGI" as disclosed herein refers to compacted graphite cast iron.

The term "SGI", or "SG", as disclosed herein refers to spheroidal graphite cast iron.

The terms "flake patches", and "patchy" as disclosed herein, relate to graphite flakes that can be formed locally in a CGI microstructure due to an insufficient amount of structure-modifying agent.

The term "treated iron sample" as disclosed herein refers to a sample of cast iron in a sample vessel, which vessel may or may not contain an agent affecting the composition of the sample. Hence, for example a treated iron sample in a tellurium cup has a reduced content of active magnesium compared to the melt from which the sample originally was taken.

The invention will now be described with references to the accompaying figures in which:
Fig. 1 defines the principles for assigning variable names in the present application;
Fig. 2 briefly outlines the effect of "extending" the thermal analysis scale by treating a cast iron sample with tellurium as neutralizing agent;
Fig. 3 shows which cast iron type the measured cooling curve actually corresponds to when carrying out a thermal analysis on a treated iron sample in a tellurium cup;
Fig. 4A discloses cooling curves corresponding to CGI, SGI, flake patchy and grey iron. which curves have been recorded in the centre of a clean cup.
Fig. 4B shows cooling curves recorded in a tellurium cup under the same conditions and for the same batch of molten cast iron as in Fig. 4A.
Figures 5A and 5B both disclose to flowcharts on how the curves obtained by the thermal analysis exemplified in Fig. 3 can be construed. Fig. 5A relates to a tellurium cup, and fig. 5B, relating to the method of the invention, relates to a set of a tellurium cup, a clean cup and a FeSi cup;
Fig. 6A and 6B show examples of sample vessels that can be used in the present method;
Fig.7 is a schematic presentation of an apparatus for controlling production of compacted graphite cast iron according to the present invention;
Fig. 8 is a micrograph showing a CGI casting that is insufficiently treated with magnesium and, accordingly contains isolated flake patches; and
Fig. 9 is a diagram showing ultimate tensile stress and 0.2% yield stress of 85-100% pearlitic cast irons as a function of nodularity and temperature.

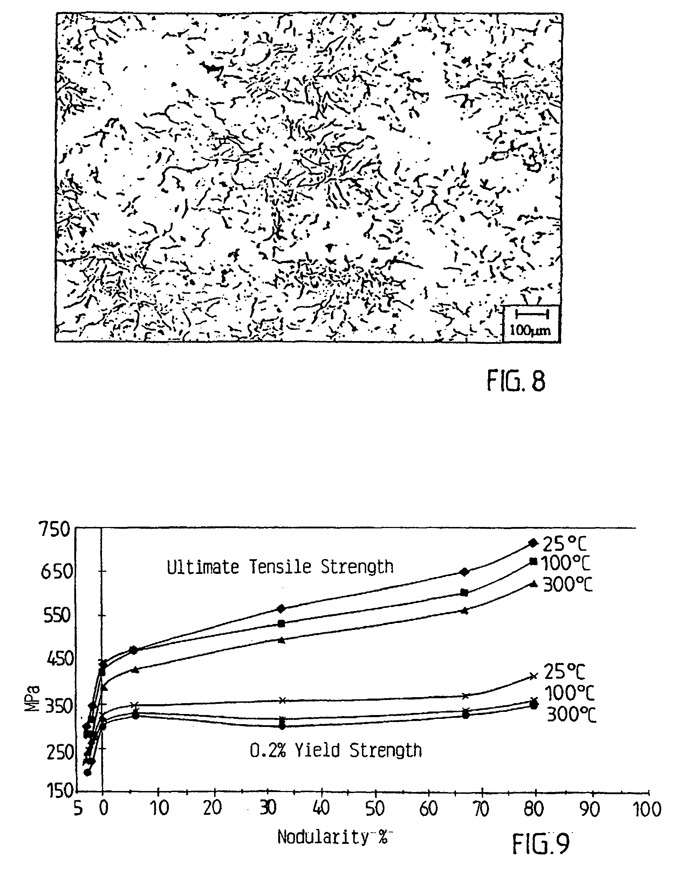

Above, the problem regarding flake patches and how such patches can impair the quality of a CGI casting has been briefly mentioned. From the practical point of view, engine designers will establish wall thickness based on the inherent strength and stiffness of the chosen material. If they are designing with CGI, they will calculate dimensions based on a minimum tensile strength of 400 MPa. However, if the foundry process is out-of-control and the castings contain flake patches, the material strength can be 20-50% less (See fig. 9). The ascast dimensions (calculated based on good CGI) will not be strong enough to withstand the operating load. The walls will crack and the engine will fail in service. The net result is an extremely costly customer liability. It is thus evident that flake patches must be avoided in CGI castings.

Traditionally, metallurgists have referred to three types of cast iron: grey (flake) cast iron; CGI; and ductile cast iron. However, when CGI is produced with insufficient shape modifying agent (magnesium) an intermediate flake patch structure can form. The centre of the eutectic cells contains flake graphite while the perimeter region of the eutectic cells contains compacted graphite particles. Surprisingly (See figure 8.) it has been shown that isolated flake patches predominate in determining the strength of the material. Even a small (10-20%) amount of flake patches will result in a loss of 20-50 % of the mechanical strength. It is therefore not sufficient to simply differentiate between CGI or grey iron: the measurement technique must have sufficient resolution to detect the onset of isolated flake patches in a predominantly compacted graphite material.

Accordingly, the present invention is directed to thermal analysis for predicting the microstructure with which a certain cast iron melt will solidify. The thermal analysis is carried out by adding molten cast iron to a sample vessel containing neutalizing agent, i.e. tellurium, whereafter the temperature is recorded during the solidification process. Optionally, cups with other coatings such as FeSi or fully inert cups (clean cups) can also be used together with the tellurium cup.

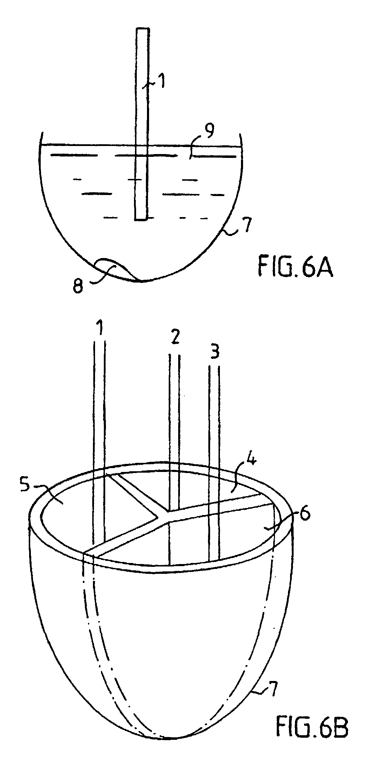

Figs. 6A and 6B present two examples of sample vessels that are used in the present invention. The sample vessel (7) in fig. 6A contains a blob (8) of tellurium, which dissolves in the molten cast iron (9). The temperature is monitored during solidification by a temperature responsive means (1) such as a thermocouple. Fig. 6B discloses a more complex vessel, which is divided into three compartments (4, 5, 6). One compartment (4) is coated with tellurium, another (5) is coated with FeSi, and the last compartment (6) does not contain any coating. Temperature-responsive means (1, 2, 3) are arranged in all three compartments. Both cups can be normal sand cups, ceramic cups or made of coated steel or other suitable materials.

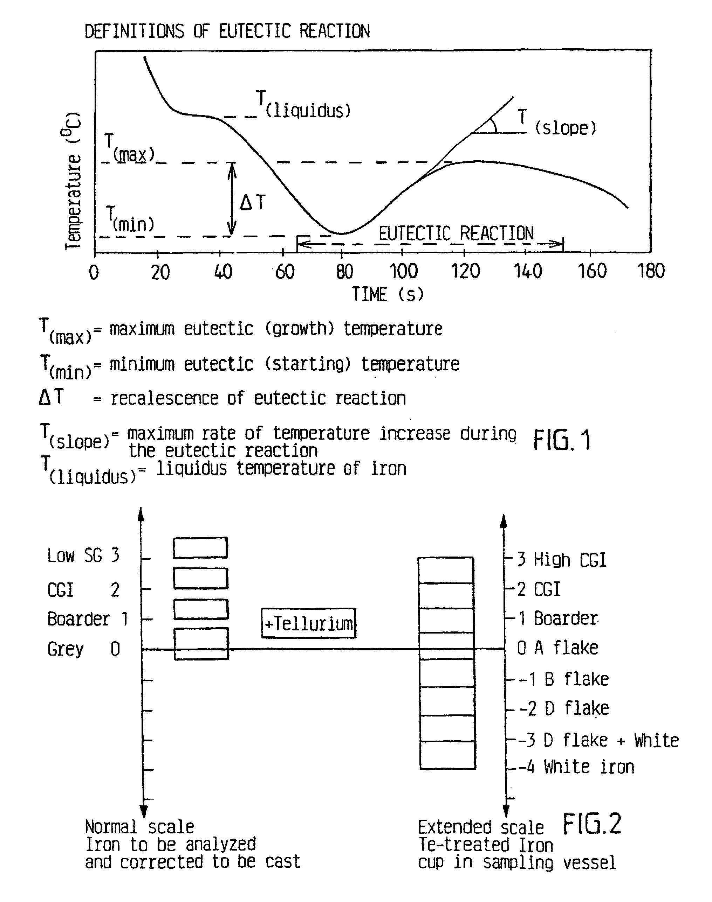

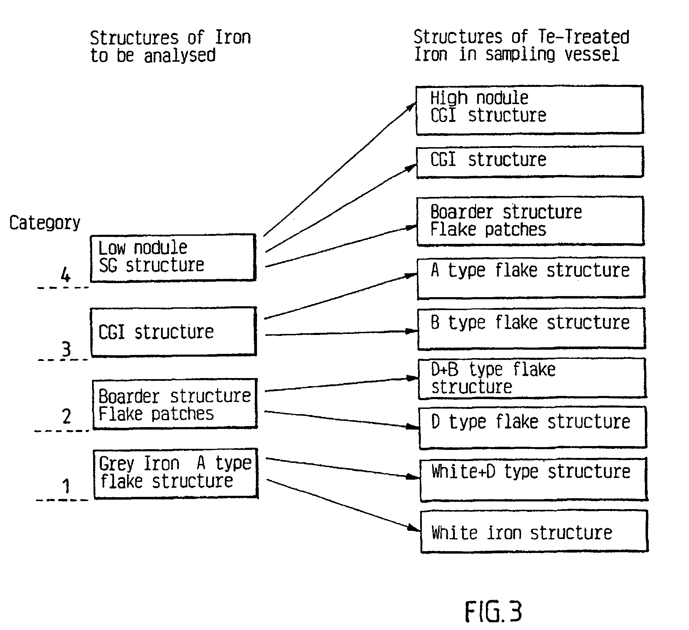

The basic principle behind the present invention is illustrated in Figs. 2 and 3. In a prediction method according to the state of the art, cooling curves corresponding to cast iron types comprised in the range from SGI to A type grey iron are recorded. In the present method, the content of structure-modifying agent is decreased by adding a predetermined amount of neutralizing agent. Hence, cooling curves corresponding to cast iron types comprised in the range from CGI to white iron are recorded instead of curves representing the actual composition of the sample.

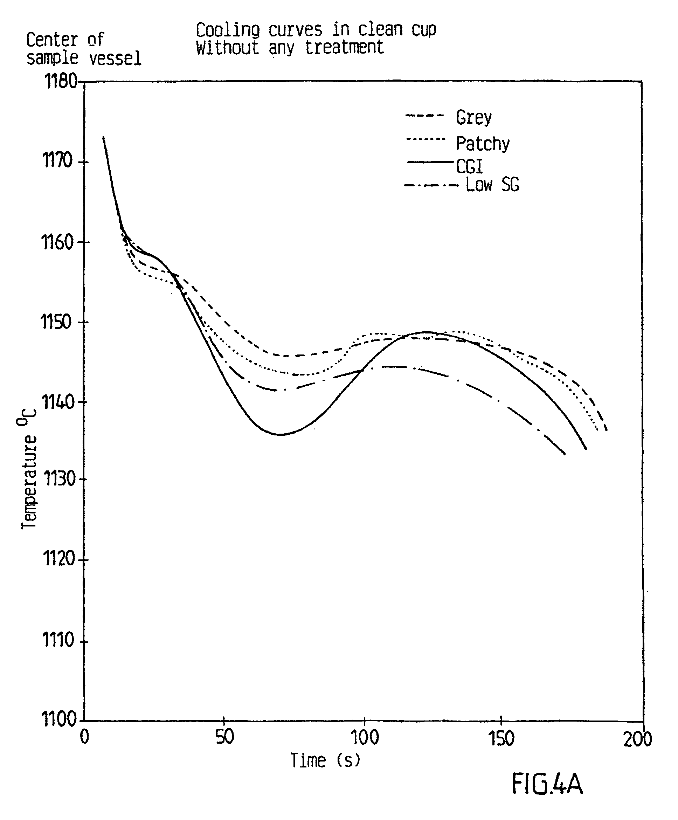

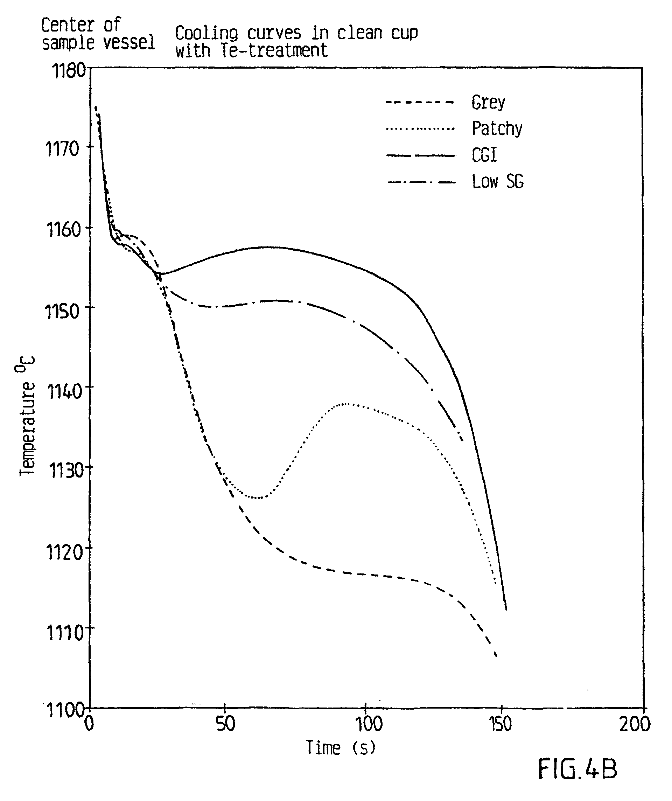

Figs. 4A and 4B illustrate the increase in sensitivity that is achieved by carrying out the present invention. Fig. 4A discloses four cooling curves corresponding to CGI, SGI, flake patchy and grey iron. No neutralizing agent has been added. The four curves can be distinguished from each other, but nevertheless they follow each other rather closely. Fig. 4B shows corresponding curves after adding a predetermined amount of a neutralizing agent. In Fig. 4A. all eutectic plateaus are comprised within the temperature range of 1140-1150°C. In contrast, the maximum growth temperatures during the eutectic reaction in Fig. 4B are comprised within the temperature range of 1115-1165°C. It is evident from the figure that a much higher accuracy is achieved by the present method compared to the state of the art.

The above mentioned predetermined amount of neutralizing agent must be effective to change grey cast iron to white iron. Tellurium is the neutralizing agent and the necessary amount of tellurium in the sample vessel is at least 0.01%(wt), preferably 0.05-0.2%(wt) of the weight of the cast iron sample.

Based on the maximum eutectic growth temperature and recalescence, results from treated iron samples can roughly be categorized into four categories. Limits between the categories are calibrated for different foundries. The following is an example of criteria for a certain foundry.

| Category | Cast Iron to be Analyzed | Maximum Growth Temperature during the Eutectic Reaction of a Treated Sample | Recalescence ΔTₜₑ |
|---|---|---|---|
| 1. | Grey iron and close to grey | <1130°C | <2°C |
| 2. | Boarder iron with flake patches | 1130°C-1150°C | >2°C |
| 3. | CGI | >1155°C | >1°C |
| 4. | Low nodule SGI | >1150°C-1155°C | <5°C |

An iron sample treated in a tellurium cup contains a lower concentration of active magnesium than corresponding untreated molten cast iron. The result of this treatment is hence that the thermal analysis is performed in a concentration region where more distinct results are obtained compared to the original concentation region.

A cooling curve recorded in a tellurium cup can therefore very clearly show whether the iron contains a sufficient amount of structure-modifying agents in order to solidify as CGI. It can also indicate whether the cast iron is overtreated with structure-modifying agents or not.

The present method also includes other thermal analysis steps which are carried out in other kinds of sample vessels, i.e. a FeSi cup and a clean cup.

Thermal analysis in a FeSi cup as well as in a clean cup gives information about both the inoculation level and the real state of cast iron. By determining the maximum and minimum eutectic temperature and maximal slope of the cooling curves the inoculation level can be calculated. If the minimum eutectic temperature of the FeSi curve is much higher than the minimum eutectic temperature of the clean curve, the inoculation level is far below saturation, and inoculants should be added. The cast iron has reached the saturation level when the two temperatures are about the same. When the cast iron is overtreated, the cooling curve from the FeSi cup has a small recalescense and a small maximal slope.

The clean cup measures the real state of iron and show the modification and inoculation level. In a certain range (assessed together with the other two cups), high recalescence (low minimum eutectic temperature and high maximum eutectic temperature) and high maximal slope point to a good CGI, while lower recalescence and lower slope indicate that the cast iron is overtreated with structure-modifying agents and/or inoculants.

Hence, thermal analysis in a tellurium cup indicates whether the molten cast iron will solidify as grey iron, flake patchy, CGI or SGI. Analyses with the FeSi cup and the clean cup and the comparisons among all three cooling curves allow sensitive and accurate determinations of the both the inoculation and the structure modification states.

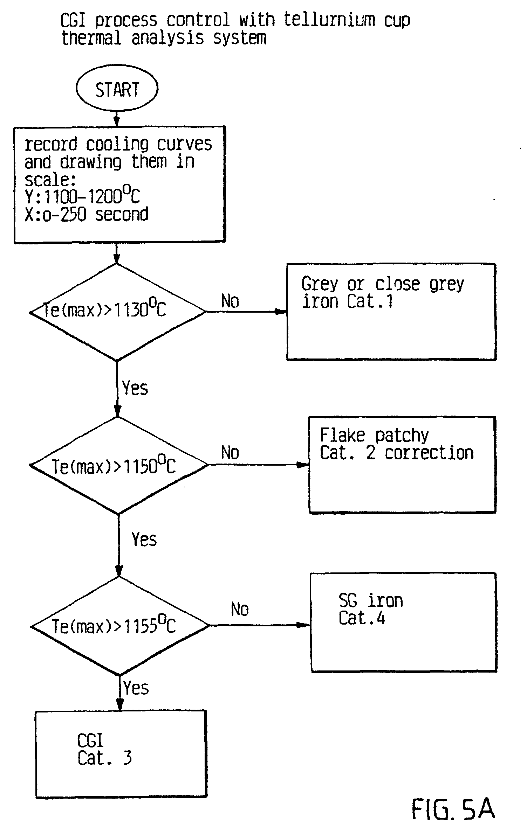

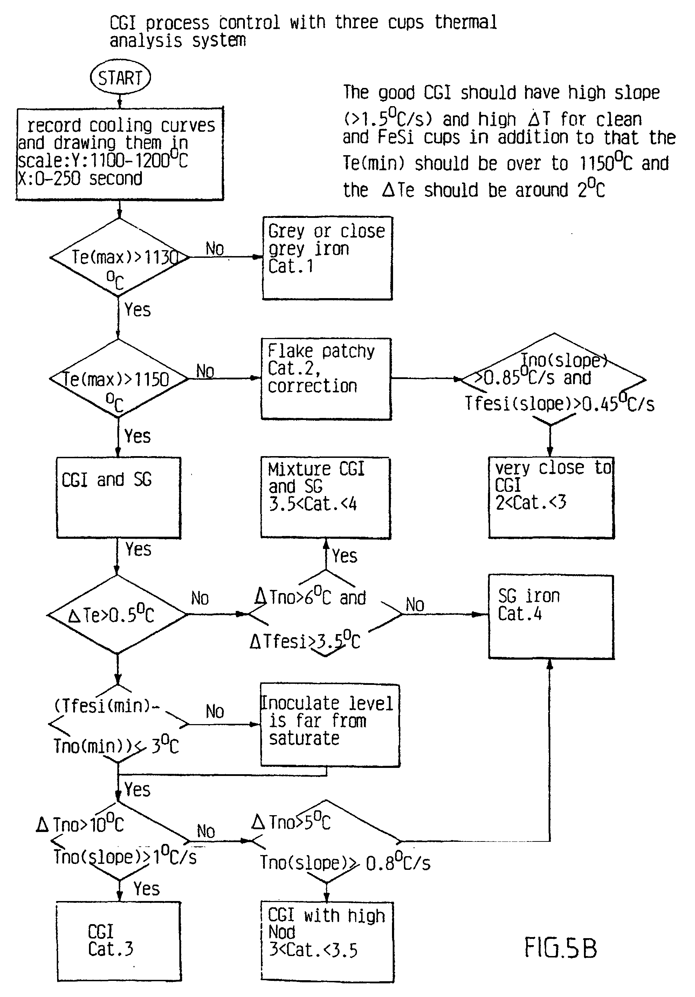

Fig. 5A outlines an example of how to construe the cooling curves that are obtained when carrying out a thermal analysis by using a tellurium cup. The first step is to determine the maximum eutectic temperature, Tₑ,of the tellurium cup curve. If it is lower than 1130°C the cast iron will solidify as grey iron. Magnesium must be added. If the maximum eutectic temperature is between 1130°C and 1150°C, the cast iron can solidify as CGI containing flake patches. A small amount of magnesium must be added. If the maximum eutectic temperature of the tellurium curve is higher than 1155°C, the sample will solidify as CGI. Otherwise, it will solidify as SGI.

Fig. 5B, relating to the method of the invention, outlines how to construe cooling curves obtained when carrying out thermal analysis in a set of three different cups, namely the tellurium cup, the clean cup and the FeSi cup. The first step is to determine the maximum eutectic temperature, Tₑ,of the tellurium cup curve. If it is lower than 1130°C the cast iron can solidify as grey iron. Magnesium must be added.

If the maximum eutectic temperature is between 1130°C and 1150°C, the cast iron will solidify as CGI containing flake patches. More information is obtained by measuring the slopes of recalescence of the cooling curves recorded in the clean cup and the FeSi cup, Tₙₒ(slope) and T_{FeSi}(slope). If Tₙₒ(slope) > 0.85°C/s and T_{FeSi}(slope) > 0.45°C/s, there is a decreased risk for flake patches, and, accordingly, a lesser amount of magnesium must be added.

If the maximum eutectic temperature of the tellurium curve is higher than 1150°C, the sample will either.solidify as CGI or SGI. In order to distinguish between CGI and SG, the recalescence of the cooling curve in the tellurium cup, ΔTₑ is determined. If this value is less than 0.5°C the corresponding recalescence values for the clean cup and the FeSi cup are determined. If ΔTₙₒ > 6°C and ΔT_{FeSi} > 3.5 °C, the cast iron will solidify as SG with a lower nodularity. Otherwise, the cast iron will solidify as SG iron.

If ΔTₑ is larger than 0.5°C, the minima of the cooling curves recorded in the FeSi cup and the clean cup are determined. If the difference between the minimum value of the Fe Si curve and the clean cup curve is larger than 3°C, the cast iron melt is not sufficiently inoculated.

Finally, the recalescence and the slope of recalescence of the clean cup curve are considered. If ΔTₙₒ > 10°C and Tₙₒ(slope)> 1 °C/s, the cast iron will solidify as CGI. If ΔTₙₒ is within the range from 5 to 10°C and Tₙₒ(slope) is within the range from 0.8 to 1 °C/s, the cast iron will solidify as CGI with high nodularity. Otherwise the cast iron will solidify as SG iron.

Following abbreviations can be found in figures 5A and 5B:
- Tₙₒ =: Eutectic temperature measured in the clean cup
- Tₑ =: - " - tellurium cup
- T_{FeSi} =: - " - FeSi cup
- Tₑ(max) =: Maximum, tellurium cup curve
- T_{FeSi}(max) =: Maximum, FeSi cup curve
- Tₙₒ(max): Maximum, clean cup curve
- Tₑ(min) =: Minimum, tellurium cup curve
- ΔTₑ =: Recalescence, tellurium cup curve
- Tₑ(slope) =: Slope of recalescence, tellurium cup curve
- Tₙₒ(slope) =: Slope of recalescence, clean cup curve
- T_{FeSi}(slope) =: Slope of recalescence, FeSi cup curve
- ΔTₙₒ =: Recalescence, clean cup curve
- ΔT_{Fe}, ΔT_{FeSi} =: Recalescence, FeSi cup
- T_{FeSi}(min) =: Minimum, FeSi cup curve
- Tₙₒ(min) =: Minimum, clean cup curve
- Cat. 1 =: grey cast iron
- Cat. 2 =: flake patchy iron
- Cat. 3 =: CGI
- Cat. 4 =: high nodularity CGI, SG

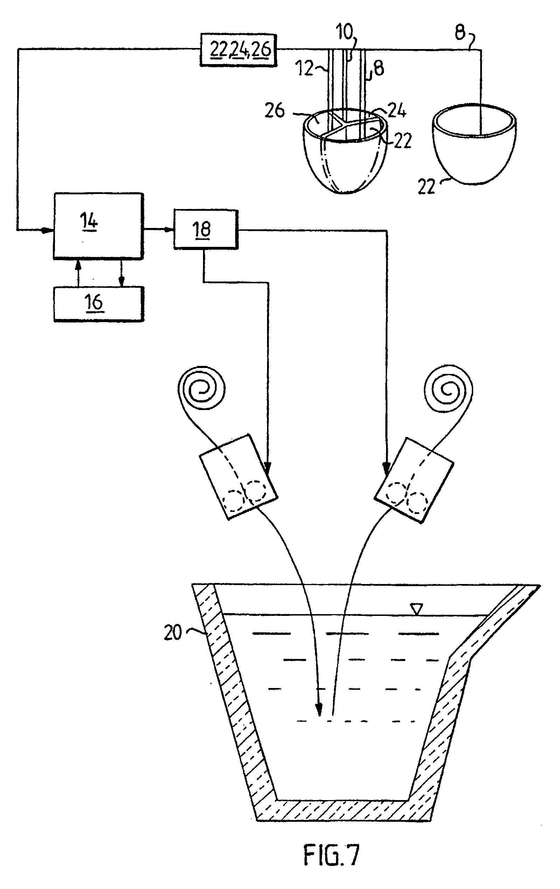

It is preferred to carry out the method by using a computer-controlled system, especially when a large number of measurements must be carried out. Such a computer controlled system is outlined in fig. 7. During the measurement of a particular sample, the three temperature responsive means 8, 10, 12 send signals to a computer means 14 in order to generate (a) cooling curve(s). The computer has access to calibration data in a ROM memory means 16 and calculates the amount of structure-modifying agents and/or inoculating agents that must be added to the melt. This amount is signalled to a means 18 for administrating structure-modifying agent and/or inoculating agent to the melt 20 to be corrected, whereby the melt is supplied with an appropriate amount of such agents. The sampling device 22 comprises a tellurium cup. Sampling device 24 comprises a FeSi cup and sampling device 26 comprises a clean cup.

## Claims

1. A method of predicting whether the microstructure in which a certain cast iron melt containing magnesium will solidify is spheroidal graphite cast iron, compacted graphite cast iron, flake patchy or grey cast iron, comprising the steps of:
a) for the chosen casting method calculating the amount A of tellurium, which amount A is sufficient to reduce the concentratio of active magnesium in a sample amount W of a cast iron melt which would solidify as grey cast iron, to a concentration of active magnesium in a sample amount W of a cast iron melt which would solidify as white iron;
b) taking a sample amount W of the cast iron melt whose microstructure is to be predicted;
c) adding the sample amount W taken in step b) to a first sample vessel containing an amount A of tellurium and then recording a cooling curve in the centre of the sample;
d) adding a sample of the cast iron melt whose microstructure is to be predicted to a second sample vessel coated with FeSi or containing a FeSi blob and recording a cooling curve in the centre of the sample;
e) adding a sample of the cast iron melt whose microstructure is to be predicted to a third sample vessel without any coating or with an inert coating and recording a cooling curve in the centre of the sample;
f) determining the following parameters of the cooling curves obtained in steps c), d) and e):
(i) the recalescence of the cooling curve recorded in step e) (ΔTno);
(ii) the recalescence of the cooling curve recorded in step c) (ΔTe);
(iii) the recalescence of the cooling curve recorded in step d) (ΔTFeSi);
(iv) the maximum eutectic temperature of the cooling curve recorded in step d) (TFeSi(max));
(v) the maximum eutectic temperature of the cooling curve recorded in step e) (Tno(max));
(vi) the maximum eutectic temperature Te(max) of the cooling curve recorded in step c);
(vii) the temperature of the local minimum of the cooling curve recorded in step c) (Te(min));
(viii) the temperature of the local minimum of the cooling curve recorded in step e) (Tno(min));
(ix) the temperature of the local minimum of the cooling curve recorded in step d) (TFeSi(min));
(x) the slope of the recalescence of the cooling curve recorded in step e) (Tno(slope));
(xi) the slope of the recalescence of the cooling curve recorded in step c) (Te(slope));
(xii) the slope of the recalescence of the cooling curve recorded in step d) (TFeSi(slope)); and
g) predicting whether whether the microstructure in which a certain cast iron melt will solidify is spheroidal graphite cast iron, compacted graphite cast iron, flake patchy, or grey cast iron by using the information obtained in step f).

2. A method according to claim 1, **characterised in that** the first sample vessel contains an amount of tellurium which is at least 0.01% of the weight of the cast iron sample.

3. A method according to claim 2, **characterised in that** the first sample vessel contains an amount of tellurium which is 0.05-0.2% of the weight of the cast iron sample.

4. A method for producing a compacted graphite iron casting, comprising the steps of:
a) carrying out the prediction method according to anyone of claims 1-3;
b) using the information obtained in step a) for calculating the amount of structure-modifying agent chosen from the group of magnesium and rare earth elements that has to be added to the melt in order to obtain compacted graphite iron;
c) adding the amount calculated in step b) to the cast iron melt; and
d) carrying out the casting operation in a per se known manner.

## Patentansprüche

1. Verfahren zur Vorhersage, ob die Mikrostruktur, in welcher eine bestimmte, Magnesium enthaltende Gusseisenschmelze erstarren wird, Kugelgraphitguss, Gusseisen mit Vermikulargraphit, uneinheitlicher Graphitlamellenoder Grauguss ist, umfassend die Schritte:
a) Berechnung der Menge A von Tellur für das ausgewählte Gießverfahren, wobei die Menge A ausreichend ist, um die Konzentration von aktivem Magnesium in einer Probenmenge W einer Gusseisenschmelze, die als Grauguss erstarren würde, zu einer Konzentration von aktivem Magnesium in einer Probenmenge W einer Gusseisenschmelze, die als weißes Gusseisen erstarren würde, zu reduzieren;
b) Entnahme einer Probenmenge W von der Gusseisenschmelze, deren Mikrostruktur vorherzusagen ist;
c) Zugabe der in Schritt b) entnommenen Probenmenge W in ein erstes Probengefäß, welches eine Menge A von Tellur enthält, und folgend Aufzeichnung einer Abkühlungskurve in dem Zentrum der Probe;
d) Zugabe einer Probe der Gusseisenschmelze, deren Mikrostruktur vorherzusagen ist, in ein zweites Probengefäß, welches mit FeSi beschichtet ist oder ein Partikel FeSi enthält, und Aufzeichnung einer Abkühlungskurve in dem Zentrum der Probe;
e) Zugabe einer Probe der Gusseisenschmelze, deren Mikrostruktur vorherzusagen ist, in ein drittes Probengefäß, ohne jegliche Beschichtung oder mit einer inerten Beschichtung, und Aufzeichnung einer Abkühlungskurve in dem Zentrum der Probe;
f) Bestimmung der folgenden Parameter der in den Schritten c), d) und e) erhaltenen Abkühlungskurven:
(i) die Rekaleszenz der in Schritt e) aufgezeichneten Abkühlungskurve (ΔTno);
(ii) die Rekaleszenz der in Schritt c) aufgezeichneten Abkühlungskurve (ΔTe);
(iii) die Rekaleszenz der in Schritt d) aufgezeichneten Abkühlungskurve (ΔTFeSi);
(iv) die maximale eutektische Temperatur der in Schritt d) aufgezeichneten Abkühlungskurve (TFeSi(max));
(v) die maximale eutektische Temperatur der in Schritt e) aufgezeichneten Abkühlungskurve (Tno(max));
(vi) die maximale eutektische Temperatur Te(max) der in Schritt c) aufgezeichneten Abkühlungskurve;
(vii) die Temperatur des lokalen Minimums der in Schritt c) aufgezeichneten Abkühlungskurve (Te(min));
(viii) die Temperatur des lokalen Minimums der in Schritt e) aufgezeichneten Abkühlungskurve (Tno(min));
(ix) die Temperatur des lokalen Minimums der in Schritt d) aufgezeichneten Abkühlungskurve (TFeSi(min));
(x) die Steigung der Rekaleszenz der in Schritt e) aufgezeichneten Abkühlungskurve (Tno(slope));
(xi) die Steigung der Rekaleszenz der in Schritt c) aufgezeichneten Abkühlungskurve (Te(slope));
(xii) die Steigung der Rekaleszenz der in Schritt d) aufgezeichneten Abkühlungskurve (TFeSi(slope)); und
g) Vorhersage, ob die Mikrostruktur, in welcher eine bestimmte Gusseisenschmelze erstarren wird, Kugelgraphitguss, Gusseisen mit Vermikulargraphit, uneinheitlicher Graphitlamellen- oder Grauguss ist, unter Verwendung der in Schritt f) erhaltenen Daten.

2. Verfahren nach Anspruch 1, **dadurch gekennzeichnet, dass** das erste Probengefäß eine Menge von Tellur enthält, welcher wenigstens 0,01 Gew.% der Gusseisenprobe entspricht.

3. Verfahren nach Anspruch 2, **dadurch gekennzeichnet, dass** das erste Probengefäß eine Menge von Tellur enthält, welcher 0,05 bis 0,2 Gew.% der Gusseisenprobe entspricht.

4. Verfahren zur Herstellung eines Gusseisenerzeugnisses aus kompaktiertem Graphitguss, umfassend die Schritte:
a) Durchführung des Vorhersage-Verfahrens gemäß einem der Ansprüche 1 bis 3;
b) Verwendung der in Schritt a) erhaltenen Daten zur Berechnung der Menge von aus der Magnesiumgruppe oder den seltenen Erden auszuwählenden strukturmodifizierendem Agens, welche zu der Schmelze zu geben ist, um kompaktierten Graphitguss zu erhalten;
c) Zugabe der in Schritt b) errechneten Menge zur Gusseisenschmelze; und
d) Durchführung des Gießvorgangs in einer an sich bekannten Weise.

## Revendications

1. Procédé permettant d'estimer si la microstructure dans laquelle une fonte à l'état fondu donnée, contenant du magnésium, se solidifie en fonte à graphite sphéroïdal, fonte à graphite compact, fonte à flocons de graphite ou fonte grise, comprenant les étapes suivantes :
a) pour le procédé de coulée choisi, calculer le montant A de tellurium suffisant pour diminuer la concentration de magnésium actif dans une quantité d'échantillon W de fonte à l'état fondu qui se solidifierait en fonte grise jusqu'à une concentration de magnésium actif dans une quantité d'échantillon W de fonte à l'état fondu qui se solidifierait en fonte blanche ;
b) prélever une quantité d'échantillon W de fonte à l'état fondu dont la microstructure est à estimer;
c) introduire la quantité d'échantillon W prélevée lors de l'étape b) dans un premier récipient contenant une quantité A de tellurium puis enregistrer une courbe de refroidissement au centre de l'échantillon;
d) introduire un échantillon de fonte à l'état fondu dont la microstructure est à estimer dans un deuxième récipient couvert de FeSi ou contenant un amas de FeSi, puis enregistrer une courbe de refroidissement au centre de l'échantillon;
e) introduire un échantillon de fonte à l'état fondu dont la microstructure est à estimer dans un troisième récipient non couvert ou couvert d'un enduit inerte, puis enregistrer une courbe de refroidissement au centre de l'échantillon;
f) déterminer les paramètres suivants des courbes de refroidissement obtenues lors des étapes c), d) et e) :
(i) la recalescence de la courbe de refroidissement enregistrée à l'étape e) (ΔTno) ;
(ii) la recalescence de la courbe de refroidissement enregistrée à l'étape c) (ΔTe) ;
(iii) la recalescence de la courbe de refroidissement enregistrée à l'étape d) (ΔTFeSi) ;
(iv) la température eutectique maximale de la courbe de refroidissement enregistrée à l'étape d) (TFeSi(max)) ;
(v) la température eutectique maximale de la courbe de refroidissement enregistrée à l'étape e) (Tno(max)) ;
(vi) la température eutectique maximale Te(max) de la courbe de refroidissement enregistrée à l'étape c) ;
(vii) la température du minimum local de la courbe de refroidissement enregistrée à l'étape c) (Te(min)) ;
(viii) la température du minimum local de la courbe de refroidissement enregistrée à l'étape e) (Tno(min)) ;
(ix) la température du minimum local de la courbe de refroidissement enregistrée à l'étape d) (TFeSi(min)) ;
(x) la pente de la recalescence de la courbe de refroidissement enregistrée à l'étape e) (Tno(slope)) ;
(xi) la pente de la recalescence de la courbe de refroidissement enregistrée à l'étape c) (Te(slope)) ;
(xii) la pente de la recalescence de la courbe de refroidissement enregistrée à l'étape d) (TFeSi(slope)) ; et
g) estimer si la microstructure dans laquelle une fonte à l'état fondu donnée va se solidifier est de la fonte à graphite sphéroïdal, de la fonte à graphite compact, de la fonte à flocons de graphite ou de la fonte grise, en utilisant les informations obtenues à l'étape f).

2. Procédé selon la revendication 1, **caractérisé en ce que** le premier récipient contient une quantité de tellurium au moins égale à 0,01% du poids de l'échantillon de fonte.

3. Procédé selon la revendication 2, **caractérisé en ce que** le premier récipient contient une quantité de tellurium comprise entre 0,05 et 0,2% du poids de l'échantillon de fonte.

4. Procédé de production de fonte à graphite compact, comprenant les étapes de :
a) mise en oeuvre du procédé d'estimation selon l'une des revendications 1 à 3 ;
b) utilisation des informations obtenues à l'étape a) pour calculer la quantité d'agent de modification de structure choisi parmi le magnésium et les terres rares qui doit être ajoutée à la coulée pour obtenir de la fonte à graphite compact ;
c) ajout de la quantité calculée à l'étape b) à la fonte ; et
d) mise en oeuvre de l'opération de coulée d'une façon connue en tant que telle.
